# EUROPEAN PATENT SPECIFICATION

(11) **EP 0 861 905 B1**
(45) Date of publication and mention of the grant of the patent: **24.11.2004**
(21) Application number: 98103109.9
(22) Date of filing: 23.02.1998
(51) Int. Cl.: C12Q 1/10, C12N 1/20, A61K 35/74, A23L 1/03, A23C 9/123

(54) **Novel lactobacilli strains useful in the treatment of disorders of the gastrointestinal system**
Neue Lactobazillus-Stämme zur Behandlung von Störungen des Gastrointestinaltraktes
Nouvelles souches de Lactobacillus utilisables au traitement de troubles du système gastrointestinal

(30) Priority: 27.02.1997 IT MI970426
(43) Date of publication of application: 02.09.1998
(73) Proprietor: Proge Farm S.r.l., 28100 Novara (IT)
(72) Inventor: Pedraglio, Gabriele, 28065 Cerano (NO) (IT)
(74) Representative: Minoja, Fabrizio, Dr.

(56) References cited:
- WO-A-95/33046
- CASTRO HP ET AL. : "Storage of lyophilized cultures of Lactobacillus bulgaricus under different relative humidities and atmospheres" APPLIED MICROBIOLOGY AND BIOTECHNOLOGY, vol. 44, 1995, pages 172-176, XP001041514
- JONG SC ET AL.: "Probiotics for humans and animals" ATCC QUARTERLY NEWSLETTER, vol. 13, no. 1, 1993, pages 1-11, XP002072789

## Description

### 1. Field of the invention

The present invention relates to novel lactobacilli useful for the treatment of disorders of the gastrointestinal system, more particularly novel strains of Lactobacillus Paracasei and Lactobacillus Salivarius, the process for the preparation thereof, pharmaceutical compositions, dietary supplements and alimentary products (for example milk, yogurt or other dairy products) containing them.

Lactobacilli are widespread in nature. A number of species are present in fruits, vegetable and dairy products (fermented milk, cheese and yogurt), whereas only some strains can be found in the gastrointestinal and urogenital tracts of mammals and insects.

The beneficial activity of lactobacilli on gastrointestinal disorders has been known since ancient times and for a long time they were ingested with milk and dairy products.

### 2. Technological background

In most cases, known lactobacilli administered through diet or in other forms are not capable of crossing the gastric acid environment, and/or of resisting bile juices, thereby getting alive and viable into the intestinal action site and developing colonies numerous enough to exert significant therapeutical effects in short times.

The treatment of the gastrointestinal disorders thus performed usually requires prolonged administration times, and is often poorly effective in the most serious, persistent cases.

US 5,032,399 discloses a specific strain of Lactobacillus acidophilus recovered from the normal intestinal flora, which has been referred to as Lactobacillus GG and has subsequently been reclassified as Lactobacillus casei subs. rhamnosus, that is reported to have beneficial effects in the treatment of gastrointestinal disorders, to be stable to acids and bile juices and to be capable of adhering to the cells of human intestinal mucosa, of producing lactic acid and of growing effectively in vitro.

Recently, the Applicant has commercialized, under the name "Enterobacilli Proge Farm", a dietary supplement containing Lactobacilli recovered from intestinal flora, which is indicated for the treatment of the gastrointestinal system disorders. Said dietary supplement, likewise other commercial products containing Lactobacilli, has to be stored at low temperatures, specifically in refrigerator (at +4°C).

### 3. Problem to be solved

Various strains of Lactobacilli recovered from the intestinal environment, when used after selection on a laboratory scale, behave as good colonizers both in vitro and in vivo, exerting their activity in a short time and with satisfactory results.

However, for them to be suitable for a commercialisation on a large scale and for the use by consumers, said bacterial strains must undergo technological treatments (such as freeze-drying, mixing with excipients, subdivision in different dose units and formulation in pharmaceutical preparations or dietary supplements) which often adversely affect said strains, easily causing them to lose their peculiar probiotic properties and their original colonization capability.

A further technological problem is the poor stability to storage of the commercial Lactobacilli strains, which usually require a fridge storage, and which anyway easily lose their activity to a great extent.

### 4. Summary

The Applicant has now unexpectedly found a process which allows to select on an industrial scale Lactobacilli strains exceedingly viable and resistant to technological treatments, which strains are useful for the therapeutical, prophylactic and anyway probiotic treatments of disorders of the gastrointestinal system in humans, said process comprising incubating in a nutritive medium a previously freeze-dried, then rehydrated mixture containing a multiplicity of Lactobacilli strains, and selecting the first proliferating bacterial colonies, which develop not later than the first 12 hours, preferably within about 10 hours of incubation, in said nutritive medium at a temperature from about 30°C to about 40°C, preferably at 37°C.

The incubation step of the mixtures of freeze-dried strains is typically carried out on a plate, in a nutritive medium added with agar, preferably on an agarized MRS medium (de Man Rogosa Sharpe broth).

The formation of the colonies is suitably evidenced under an optical microscope, in particular by observing the reverse of the plate.

The mixture to be freeze-dried comes from the normal intestinal bacterial flora from an healthy human subject, and is typically obtained from biological samples containing a great number of bacterial species (for example intestinal mucosa, faeces), recovered from human intestinal environment or from its contents by conventional techniques.

Before undergoing the process of the present invention, these samples are usually subjected to suitable pre-treatments according to conventional methods (for example physical pre-treatments, such as homogenization, and/or dilution in a suitable diluent, for example water).

The present process further comprises one or more pre-selection steps by incubation in a known nutritive medium selective for Lactobacilli, in the incubation conditions known for the development of Lactobacilli, from which bacterial mixtures highly enriched in Lactobacilli are obtained.

Said pre-selection step is preferably carried out before freeze-drying, on the biological sample preferably pre-treated as described above, to obtain bacterial mixtures which are subsequently subjected to freeze-drying and to the other selection steps according to the present invention, this step being typically effected in a liquid medium, for example a liquid MRS medium optionally added with other nutrients or useful substances, as specified hereinafter.

Further objects of the present invention are Lactobacilli strains and the bacterial cultures selected through the present process, the preparations or formulations containing the present Lactobacilli in combination with a pharmaceutically or anyway physiologically acceptable carrier, such as therapeutical and/or prophylactic compositions, dietary supplements, medical devices, alimentary products.

### 5. Detailed disclosure

The present process allows to select highly viable Lactobacilli, suitable for formulation on an industrial scale as pharmaceutical preparations or dietary supplements, which proved to be highly effective and advantageous in the treatment of various disorders of the gastrointestinal system.

According to an embodiment of the present invention, the mixtures of bacterial strains to be subjected to the present process come from samples of human faeces. According to another embodiment of the present invention, mixtures containing a great number of Lactobacilli belonging to human microintestinal flora are freeze-dried, rehydrated, incubated in a nutritive medium, the selected strains being incubated as described above, then further subjected to one or more of the following selection steps, preferably to all of them, in any order:
a) incubation in a nutritive medium containing bile, in a concentration of at least 0.1% weight/volume, and selection of the strains proliferating in said medium;
b) incubation in a nutritive acid medium, having pH lower than or equal to 5.5 (more preferably lower than 3.5), and selection of the strains proliferating in said medium;
c) incubation in a nutritive medium containing cells of the intestinal mucosa of the subject to treat, in particular humans, and selection of the bacteria capable of adhering to the cells at an average rate corresponding to the adhesion of at least 50 bacteria per each intestinal cell in an incubation time up to 5 minutes;
d) incubation of the strains in a nutritive medium and selection of the bacteria which produce at least 3.5 mEq of lactic acid per 10¹⁰ CFU (Colony Forming Units) at 37°C in a time of 24 hours;
e) incubation of the strains in a nutritive medium, and selection of the bacterial strains which proliferate at 37°C with a generation time of less than one hour, more preferably of about 45 minutes.

The selection steps indicated above as a) to e) are typically carried out in the operative conditions described in US 5,032,399 and in the corresponding EP 199,535, with the difference that mixtures of microorganisms from a previous selection, deriving from the collection of microorganisms capable of differentiating in a nutritive medium incubated with previously freeze-dried and rehydrated strains, are subjected to steps a) to d).

More specifically, the present process comprises the following steps:
1) incubation of a mixture of microorganisms containing a multiplicity of Lactobacilli in a medium selective for Lactobacilli, and collection of the thus developed bacterial cells;
2) freeze-drying of the bacterial cells obtained from the previous step, preferably in the presence of one or more additives for freeze-drying;
3) rehydration of the freeze-dried mixture from the previous step;
4) incubation of the resulting freeze-dried and rehydrated mixture in a nutritive medium, preferably in a medium selective for Lactobacilli, and collection of the cells developed not later than the first 12 hours, preferably within about 10 hours of incubation at 37°C in said nutritive medium.

The cultures of Lactobacilli recovered according to the present process can be sometimes contaminated by traces of bacterial strains belonging to other species from microintestinal flora, without departing from the scope of the present invention.

The medium selective for Lactobacilli is, for example, an MRS culture broth (de Man Rogosa Sharpe broth, Oxoid), commercialized by DIFCO and described in J. Applied Bacteriol. (1960), 23, 130, having the composition reported in the above cited US 5,032,399 and EP-B199,535, or other selective medium known for this purpose, optionally added with other nutrients or other substances. For example an MRS medium added with bile salts (for example in a 0.5% weight/volume concentration on the total of the broth) can be used.

The incubation in a medium selective for Lactobacilli according to step 1) is typically carried out in a liquid medium.

The incubation of the freeze-dried, rehydrated bacterial strains, as in step 4) is typically effected on agarized plates, preferably in a selective medium for Lactobacilli, although, alternatively, it can be effected in a different nutritive medium conventionally known for their development.

The above cited incubation steps of lactobacilli are typically carried out at a temperature from 30°C to 40°C, preferably at 37°C, preferably under mildly anaerobic atmosphere.

The freeze-drying is preferably carried out in the presence of additives known as freeze-drying protectives, for example powder skimmed milk (in amounts for example of about 10-12% by weight on the basis of the Lactobacilli to be freeze-dried), saccharose, glycerol, or anyway operating according to alternative, conventional freeze-drying techniques.

The present process allows to select Lactobacilli, such as the strains deposited at the Institut Pasteur, as reported in the following, having high resistance to the technological treatments, particularly high viability and high stability to storage at temperatures of about +15°C/+20°C, and moreover, of one or more of the following advantageous and surprising characteristics: ability to produce lactic acid in a substantially pure stereomeric L-(+) form, or anyway in an amount by far higher than the isomer D(-), resistance to various antibiotics, in particular insensitiveness to tetracyclins but sensitivity to other antibiotics, in particular rifampicin and erythromycin.

According to a further advantageous characteristic, the lactobacilli obtained according to the present process are not capable of transferring their resistance to tetracyclins to other microorganisms, including the pathogenic ones.

In particular, the lactobacilli according to the present invention have the further features:
- stability to acids;
- stability to bile juices;
- capability of adhering to the cells of the intestinal mucosa;
- capability of growing effectively in vitro.

The capability of producing only, or anyway in a high prevalence, L-(+)-Lactic acid, i.e. the L(+) isomer, which is not metabolized by the body, and can remain for a long time in the intestinal lumen, where it exerts its effects, is extremely advantageous, in that the lactic acid produced by lactobacilli is at least partly responsible for the beneficial effects of the colonization of the gastrointestinal tract by lactobacilli, thus opposing pathogens presumably through a lowering of the pH of the intestinal environment.

The sensitivity to some types of antibiotics evidenced for the lactobacilli according to the present invention is an important, advantageous feature. It is in fact known that "cross-resistance" phenomena to antibiotics can occur through migration of plasmidic material from one microorganism to another. Therefore, the administration of Lactobacilli resistant to all of the antibiotics is potentially dangerous for the body, in that pathogens strains resistant to all of the antibiotics could be selected.

It is therefore important that the Lactic bacterium administered for the treatment of disorders of the gastrointestinal system be sensitive to at least some antibiotics, so as to have suitable therapeutical weapons available, should the above mentioned cross-resistance occur.

Contrary to the Lactobacilli recovered by the present process, various known Lactobacilli strains, among which the Lactic bacterium GG disclosed in US 5,032,399, turned out to be insensitive to all of the antibiotics and thus disadvantageous from this point of view.

The selection according to the process of the invention can be operated periodically so as to isolate and use strains with different lysotypia, keeping however probiotic characteristics , such as:
- ability to reach the action site remaining integer;
- ability to settle at the gastrointestinal level;
- production of L-(+)-Lactic acid.

The possibility of selecting strains with different lysotypia allows to assure the productivity and effectiveness of the fermentation process and/or a safe colonization in the intestine even in the presence of phagi, which are capable of phagocytizing and destroying lactobacilli both in the fermentation environment and in the intestinal lumen, thus jeopardizing the production of lactobacilli or the therapeutical or dietetic treatments making use of them.

Moreover, the Lactobacilli strains of the invention are unexpectedly endowed with a higher stability compared with those obtained by the known processes, as they can be stored for long times at temperatures above 4°C, in particular at about +15°C/+20°C. For example, after about 18-24 months at about 22°C, the decrease in viable cells observed is only of 1 logarithmic unit.

Thanks to this property, the preparations of the invention show amounts of lactobacilli alive of only about 1 logarithm less of cells per unitary dose, compared with the number of cells alive at time zero, even after months from the commercialisation, contrary to what observed for various commercial compositions of the prior art, which show a high decrease in the alive cells compared with those specified at the time of the packaging, and which therefore are often poorly effective from the therapeutical point of view.

In fact, the treatment with Lactobacilli according to the present invention yields favourable results, such as the nearly complete remission of diarrhoea, even when severe and obstinate, due for example to antitumour radiotherapy, in short times i.e. about 1-2 days, which is markedly less than the time required with the lactobacilli of the prior art. The rapidity of action can be at least partly ascribed to the high storage stability shown by the Lactobacilli of the invention.

More precisely, the process of the invention allowed to select novel Lactobacilli strains belonging to the Paracasei and Salivarius species, in particular 3 strains deposited under the Budapest Treaty at the "Collection Nationale de Cultures de Microorganismes" (CNCM) of the Institut Pasteur (25, Rue du Docteur Roux, 75724 PARIS CEDEX 15), under the following accession numbers:
- Lactic bacterium PF1S, deposited on April 3, 1996, under the accession number CNCM I-1687, with the taxonomic designation Lactobacillus Paracasei;
- Lactic bacterium PF2P, deposited on April 3, 1996, under the accession number CNCM I-1688, with the taxonomic designation Lactobacillus Paracasei;
- Lactic bacterium PF1S1, deposited on December 6, 1996, under the accession number CNCM I-1794, with the taxonomic designation Lactobacillus Salivarius.

The three Lactobacilli strains Paracasei and Salivarius above identified by their accession numbers produce exclusively L(+) Lactic acid, are sensitive to erythromycin and rifampicin and insensitive to tetracyclines, are stable to acids, resisting at pH 3.5 for 30 minutes at 40°C.

In particular, the L. Paracasei strains, particularly the Lactic bacterium PF2P CNCM I-1688, are insensitive to aztreonam, cefotixina, vancomycin and tetracycline, and sensitive to erythromycin, rifampicin and cefotaxime, and they ferment ribose; and Salivarius strains, such as the PF1S1 CNCM I-1794 strain, are insensitive to cefotaxime and tetracycline, and sensitive to erythromycin and rifampicin.

The lactobacilli according to the present invention can be administered as such or they can be added to a pharmaceutically or anyway physiologically acceptable carrier, to obtain various types of preparations or formulations, such as pharmaceutical compositions, dietary supplements or alimentary products, in particular dairy products (such as milk, yogurt), which are a further object of the present invention.

The present preparations or formulations contain at least one Lactic bacterium according to the present invention, preferably mixtures of at least one Lactic bacterium of the species Paracasei or Salivarius, in particular in effective amounts for the therapeutical, prophylactic or probiotic treatments desired and can be prepared, for example, according to conventional techniques.

The carrier can contain excipients, additives, diluents or other useful substances, provided they are pharmacologically or physiologically acceptable.

For the treatment of the disorders of the gastrointestinal system according to the invention, lactobacilli are typically administered orally, preferably as Paracasei and Salivarius mixtures.

The daily effective amount for humans is generally comprised from 10⁷ to about 2 x 10⁹ Lactobacilli daily, for an adult (weighing on the average 50-70 Kg), or from 10⁶ to 10⁸ Lactobacilli for infants.

Typical preparations for the treatment of disorders of the gastrointestinal system according to the present invention contain for example 10⁶ to 3 x 10⁹ UFC of Lactobacilli for unitary dose.

Preferably, the preparations according to the invention contain lactobacilli in the freeze-dried form.

For this purpose, aqueous mixtures containing lactobacilli, and preferably at least one freeze-drying additive, are typically freeze-dried in the conditions reported above for the process of selection of lactobacilli according to the invention, or according to known procedures.

Preferably, for the purposes of the invention, lactobacilli, typically in freeze-dried form, are combined with excipients or additives selected from: vitamins (such as vitamins of the group B, for example vitamin B1 and B2, vitamin C and mixtures thereof), hydrocarbons (for example sweeteners, such as saccharose, and maltodextrins) and silicium dioxide, preferably in admixture, for example mixtures of vitamins B1, B2, C, saccharose, maltodextrins and silicium dioxide.

Vitamins are present in amounts depending on the dosage typical of the vitamin, for example about 0.1-5 mg of vitamin B1 or B2 for 10⁹ Lactobacilli and about 5-20 mg of Vitamin C for 10⁹ Lactobacilli; hydrocarbons are present for example in amounts of 500-1000 mg for 10⁹ Lactobacilli (for example about 500-800 mg of saccharose for 10⁹ Lactobacilli, preferably combined with about 100-300 mg of maltodextrins for 10⁹ Lactobacilli); silicium dioxide is present for example in amounts of about 0.5-5.0 mg for 10⁹ Lactobacilli.

Preferably, in the final product, the freeze-dried product containing the Lactobacilli is packaged in sachets in paper/aluminium/polyethylene foil.

The lactobacilli of the invention are valuable for the treatment of various disorders of the gastrointestinal tract, for example those due to alterations of the equilibrium of the intestinal flora, in particular diarrhoea and constipation, for example specific and aspecific diarrhoea (specially diarrhoea following the use of antibiotics), chronic intestinal inflammations, colitis, flatulence, heartburn, gastroenteritis, aphthous stomatitis, peristaltic disorders, other conditions of gastrointestinal impaired function and/or dysmicrobism consequent to surgery, kidney or liver disorders, radiotherapy, dietary unbalances, emotional stress, ageing or immune system disorders.

The Lactobacilli of the invention are moreover effective in the treatment of the irritable bowel. This is a particularly unexpected result in that, whereas lactobacilli are known to colonize mainly the distal intestine, they are not known to be capable of colonize colon in large amounts.

Some exemplary embodiments of the invention are reported in the following.

### EXAMPLE

Composition for the oral use, in freeze-dried form, contained in sachets of paper/aluminium/polyethylene foil, each containing:
- Lactobacillus Paracasei PF2P, in amounts of at least 10⁹ UFC;
- Lactobacillus Salivarius PF1S1, in amounts of at least 10⁹ UFC;

| | |
|---|---|
| Vitamin B1 | mg 0.6; |
| Vitamin B2 | mg 0.5; |
| Vitamin C | mg 20.0; |
| Saccharose | mg 1417.8; |
| Maltodextrins | mg 400.0; |
| Cream flavour | mg 50.0; |
| Silicium dioxide | mg 5.0; |

or
- Lactobacillus Paracasei PF1S, in amounts of at least 10⁹ UFC;
- Lactobacillus Salivarius PF1S1, in amounts of at least 10⁹ UFC;

| | |
|---|---|
| Vitamin B1 | mg 0.6; |
| Vitamin B2 | mg 0.5; |
| Vitamin C | mg 20.0; |
| Saccharose | mg 1417.8; |
| Maltodextrins | mg 400.0; |
| Cream flavour | mg 50.0; |
| Silicium dioxide | mg 5.0. |

The following tables show the fermentation profiles of the three Lactobacilli strains deposited at the Institut Pasteur, obtained by means of the API 50CHL system.

**TABLE 1**

| |
|---|
| The strain of L. Paracasei PF1S-CNCM I-1687 ferments the following hydrocarbons: |
| L-Arabinose, Ribose, Galactose, D-Glucose, D-Fructose, D-Mannose, L-Sorbose, Rhamnose, Dulcitol, Inositol, Mannitol, Sorbitol, α-Methyl-D-Mannoside, α-Methyl-D-Glucoside, N-Acetylglucosamine, Amygdalin, Arbutin, Esculin, Salicin, Cellobiose, Maltose, Lactose, Melibiose, Saccharose, Threalose, Inulin, Melicitose, β-Gentiobiose, D-Turanose, D-Tagatose. |

**TABLE 2**

| |
|---|
| L. Paracasei PF2P-CNCM I-1688 ferments the following hydrocarbons: |
| L-Arabinose, Ribose, Galactose, D-Glucose, D-Fructose, D-Mannose, L-Sorbose, Rhamnose, Dulcitol, Inositol, Mannitol, Sorbitol, α-Methyl-D-Mannoside, α-Methyl-D-Glucoside, N-Acetylglucosamine, Amygdalin, Arbutin, Esculin, Salicin, Cellobiose, Maltose, Lactose, Melibiose, Saccharose, Trealose, Inulin, Melicitose, a-Gentiobiose, D-Turanose, D-Tagatose. |

**TABLE 3**

| |
|---|
| L. Salivarius strain PF1S1-CNCM I-1794 ferments the following hydrocarbons: |
| Galactose, D-Glucose, D-Fructose, D-Mannose, Mannitol, Sorbitol, N-Acetylglucosamine, Maltose, Lactose, Melibiose Saccharose, Trealose, D-Raffinose, Xylitol, D-Arabitol. |

## Claims

1. A process for the selection of Lactobacilli strains useful in the treatment of disorders of the gastrointestinal tract in humans, comprising the incubation in a nutritive medium of a mixture containing a multiplicity of Lactobacilli strains, coming from the normal human intestinal bacterial flora, previously freeze-dried and then rehydrated, followed by the selection of the colonies which develop not later than the first 12 hours of incubation in said nutritive medium at a temperature from 30°C to 40°C.

2. A process according to claim 1, comprising the following steps:
1) incubation of a mixture of microorganisms containing a multiplicity of Lactobacilli in a selective medium for Lactobacilli, and collection of the bacterial cells thus developed;
2) freeze-drying of the bacterial cells from the previous step, in the presence of one or more freeze-drying additives;
3) rehydration of the freeze-dried mixture from the previous step;
4) incubation of the resulting freeze-dried and rehydrated mixture, in a medium for selective Lactobacilli or in other nutritive mediums, and collection of the cells developed not later than the first 12 hours of incubation at 37°C in said nutritive medium.

3. A process according to claim 1 or 2, in which the collection of the cells developed after incubation of the above freeze-dried and rehydrated mixture is carried out within 10 hours of incubation at 37°C.

4. A process according to claim 1 or 2, in which the incubation is carried out in an MRS culture broth.

5. A process according to claims 1 to 4, in which the incubation of the above freeze-dried and rehydrated mixture is carried out on plates, in a nutritive medium added with agar.

6. A process according to claim 1 or 2, in which the freeze-drying is carried out in the presence of freeze-drying additives selected from powder skimmed milk, saccharose and glycerol.

7. A process according to claim 1 or 2, in which the incubation of the above freeze-dried and rehydrated strains is followed by one or more of the following selection steps, carried out in any order:
a) incubation in a nutritive medium containing bile, in concentrations of at least 0.1% weight/volume, and selection of the strains proliferating in said medium;
b) incubation in a nutritive acid medium, having pH lower than or equal to 5.5, and selection of the strains proliferating in said medium;
c) incubation in a nutritive medium containing cells of the intestinal mucosa of the subject to treat, and selection of the bacteria capable of adhering to the cells at an average rate corresponding to the adhesion of at least 50 bacteria for each intestinal cell in an incubation time lower than or equal to 5 minutes;
d) incubation of the strains in a nutritive medium and selection of the bacteria which produce at least 3.5 mEq of lactic acid per 10¹⁰ UFC (Colony Forming Units) at 37°C in a time of 24 hours;
e) incubation of the strains in a nutritive medium, and selection of the bacterial strains which proliferate within a generation time at 37°C of less than one hour.

8. A culture selected from the following cultures deposited at the CNCM collection of the Institut Pasteur:
- Lactobacillus Paracasei, CNCM I-1687;
- Lactobacillus Paracasei, CNCM I-1688;
- Lactobacillus Salivarius, CNCM I-1794.

9. A preparation for the treatment of disorders of the gastrointestinal system in humans, containing an effective amount of at least one Lactic bacterium strain as defined in claim 8, in combination with a pharmaceutically or physiologically acceptable carrier.

10. A preparation according to claim 9, selected from pharmaceutical compositions, dietary supplements, and alimentary products.

11. A preparation according to claim 10, for the oral use.

12. A preparation according to claim 9, in which said Lactic bacterium strain is in the freeze-dried form.

13. A preparation according to claim 9, selected from pharmaceutical compositions and dietary supplements, in which lactobacilli are combined with excipients or additives selected from vitamins, hydrocarbons and silicium dioxide.

14. A preparation according to claim 9, containing vitamin B1 and B2, vitamin C and mixtures thereof, saccharose, maltodextrins and silicium dioxide.

15. The use of a culture containing a Lactic bacterium strain as defined in claim 8 for the preparation of a medicament for the treatment of disorders of the gastrointestinal system in humans.

16. The use according to claim 15, in which said disorders are selected from the group consisting of specific and non-specific diarrhoea, constipation, colitis, flatulence, heartburn, gastroenteritis, aphthous stomatitis and irritable bowel syndrome.

## Patentansprüche

1. Verfahren zur Auswahl von Lactobacillus-Stämmen, die nützlich in der Behandlung von Störungen des Gastrointestinaltraktes beim Menschen sind, das umfasst: Das Inkubieren in einem Nährmedium einer Mischung, die eine Mehrzahl von Lactobacillus-Stämmen enthält, die in einer normalen humanen Instestinalbakterienflora vorkommen, die vorher gefriergetrocknet und anschließend rehydratisiert wurden, gefolgt durch die Auswahl von Kolonien, die sich nicht später als in den ersten zwölf Stunden der Inkubation in dem genannten Nährmedium bei einer Temperatur von 30°C bis 40°C entwickeln.

2. Verfahren nach Anspruch 1 , das die folgenden Schritte umfasst:
1) Inkubation einer Mischung von Mikroorganismen, die eine Mehrzahl von Lactobazillen enthalten, in einem Selektivmedium für Lactobazillen und Isolieren der so entwickelten Bakterienzellen;
2) Gefriertrocknen der Bakterienzellen aus dem vorhergehenden Schritt in Gegenwart von einem oder mehreren Gefriertrocknungsadditiven;
3) Rehydratisierung der gefriergetrockneten Mischung aus dem vorherigen Schritt;
4) Inkubation der resultierenden gefriergetrockneten und rehydratisierten Mischung in einem Medium für selektive Lactobazillen oder in einem anderen Nährmedium und Isolieren der Zellen, die sich nicht später als in den ersten zwölf Stunden der Inkubierung bei 37°C in dem genannten Nährmedium entwickeln.

3. Verfahren nach Anspruch 1 oder 2, worin das Isolieren der Zellen, die sich nach der Inkubation der oben erwähnten gefriergetrockneten und rehydratisierten Mischung entwickelt haben, innerhalb der ersten zehn Stunden der Inkubation bei 37°C durchgeführt wird.

4. Verfahren nach Anspruch 1 oder 2, worin die Inkubation in einer MRS-Kulturbrühe durchgeführt wird.

5. Verfahren nach Ansprüchen 1 bis 4, worin die Inkubation der obigen gefriergetrockneten und rehydratisierten Mischung auf Platten in einem Nährmedium, das mit Agar versetzt ist, durchgeführt wird.

6. Verfahren nach Anspruch 1 oder 2, worin das Gefriertrocknen in Gegenwart von Gefriertrocknungsadditiven durchgeführt wird, die ausgewählt werden aus Magermilchpulver, Saccharose und Glycerin.

7. Verfahren nach Anspruch 1 oder 2, worin die Inkubation der oben erwähnten gefriergetrockneten und rehydratisierten Stämme gefolgt wird von einem oder mehreren der folgenden Auswahlschritte, die in beliebiger Reihenfolge durchgeführt werden:
a) Inkubation in einem Nährmedium, das Galle enthält in Konzentrationen von mindestens 0,1 % Gewicht/Volumen und Auswahl der Stämme, die sich in dem genannten Medium vermehren;
b) Inkubation in einem sauren Nährmedium, das einen pH von gleich oder weniger als 5,5 aufweist und Auswahl der Stämme, die sich in dem genannten Medium vermehren;
c) Inkubation in einem Nährmedium, das Zellen der Intestinalschleimhaut des zu behandelnden Subjektes enthält und Auswahl der Bakterien, die fähig sind, an die Zellen mit einer Durchschnittsrate zu haften, die der Haftung von mindestens 50 Bakterien für jede Intestinalzelle entspricht, während einer Inkubationsdauer, die kleiner oder gleich 5 Minuten ist;
d) Inkubation der Stämme in einem Nährmedium und Auswahl der Bakterien, die mindestens 3,5 mEq Milchsäure pro 10¹⁰ UFC (Kolonie-formende Einheiten) bei 37°C in einer Zeit von 24 Stunden erzeugen;
e) Inkubation von Stämmen in einem Nährmedium und Auswahl der Bakterienstämme, die sich bei 37°C innerhalb einer Bildungsdauer von weniger als einer Stunde vermehren.

8. Kultur ausgewählt aus den folgenden Kulturen, die bei der CNCM-Sammlung des Instituts Pasteur hinterlegt sind:
- Lactobacillus Paracasei, CNCM I-1687;
- Lactobacillus Paracasei, CNCM I-1688;
- Lactobacillus Salivarius, CNCM I-1794.

9. Präparat zur Behandlung von Störungen des Gastrointestinalsystems beim Menschen, das eine wirksame Menge von mindestens einem Milchsäurebakterienstamm, wie in Anspruch 8 definiert, in Kombination mit pharmazeutisch oder physiologisch verträglichen Trägern enthält.

10. Präparat nach Anspruch 9, ausgewählt aus pharmazeutischen Zusammensetzungen, Nahrungsergänzungsmitteln und Lebensmittelprodukten.

11. Präparat nach Anspruch 10 für die orale Anwendung.

12. Präparat nach Anspruch 9, worin der genannte Milchsäurebakterienstamm in gefriergetrockneter Form vorliegt.

13. Präparat nach Anspruch 9, ausgewählt aus pharmazeutischen Zusammensetzungen und Nahrungsergänzungsmitteln, worin die Lactobazillen kombiniert sind mit Exzipienten oder Additiven, die ausgewählt sind aus Vitaminen, Kohlenwasserstoffen und Siliciumdioxid.

14. Präparat nach Anspruch 9, das Vitamin B1 und B2, Vitamin C und Mischungen davon, Saccharose, Maltodextrine und Siliciumdioxid enthält.

15. Verwendung einer Kultur, die einen Milchsäurebakteriumstamm, wie in Anspruch 8 definiert, enthält, für die Herstellung eines Medikamentes für die Behandlung von Störungen des Gastrointestinalsystems beim Menschen.

16. Verwendung nach Anspruch 15, worin die genannten Störungen ausgewählt werden aus der Gruppe, die besteht aus spezifischen und nicht spezifischen Diarrhöen, Verstopfungen, Colitis, Blähungen, Sodbrennen, Gastroenteritis, aphthöse Stomatitis und Reizdarm.

## Revendications

1. Procédé pour la sélection de souches de Lactobacilles utiles dans le traitement des troubles de l'appareil gastro-intestinal chez les humains, comprenant l'incubation dans un milieu nutritif d'un mélange contenant une multiplicité de souches de Lactobacilles, provenant de la flore bactérienne intestinale humaine normale, préalablement lyophilisées, puis réhydratées, en faisant suivre par la sélection des colonies qui se développent durant les 12 premières heures d'incubation dans ledit milieu nutritif à une température de 30°C à 40°C.

2. Procédé selon la revendication 1, comprenant les étapes suivantes :
1) incubation d'un mélange de microorganismes contenant une multiplicité de Lactobacilles dans un milieu sélectif pour Lactobacilles, et récupération des cellules bactériennes ainsi développées ;
2) lyophilisation des cellules bactériennes de l'étape précédente, en présence d'un ou plusieurs additif(s) de lyophilisation ;
3) réhydratation du mélange lyophilisé de l'étape précédente ;
4) incubation du mélange lyophilisé et réhydraté résultant, dans un milieu pour Lactobacilles sélectives ou dans d'autres milieux nutritifs, et récupération des cellules développées durant les 12 premières heures d'incubation à 37°C dans ledit milieu nutritif.

3. Procédé selon la revendication 1 ou 2, dans lequel la récupération des cellules développées après incubation du mélange lyophilisé et réhydraté est effectuée dans la plage de 10 heures d'incubation à 37°C.

4. Procédé selon la revendication 1 ou 2, dans lequel l'incubation est effectuée dans un bouillon de culture MRS.

5. Procédé selon les revendications 1 à 4, dans lequel l'incubation du mélange lyophilisé et réhydraté est effectuée sur des plaques, dans un milieu nutritif additionné d'agar agar.

6. Procédé selon la revendication 1 ou 2, dans lequel la lyophilisation est effectuée en présence d'additifs de lyophilisation choisis entre le lait écrémé en poudre, le saccharose et le glycérol.

7. Procédé selon la revendication 1 ou 2, dans lequel l'incubation des souches lyophilisées et réhydratées est suivie par une ou plusieurs des étapes de sélection suivantes, effectuées dans n'importe quel ordre :
a) incubation dans un milieu nutritif contenant de la bile, à des concentrations d'au moins 0,1% en poids/volume, et sélection des souches proliférant dans ledit milieu ;
b) incubation dans un milieu nutritif acide, ayant un pH inférieur ou égal à 5,5, et sélection des souches proliférant dans ledit milieu ;
c) incubation dans un milieu nutritif contenant des cellules de la muqueuse intestinale du sujet à traiter, et sélection des bactéries capables d'adhérer aux cellules dans une proportion moyenne correspondant à l'adhérence d'au moins 50 bactéries pour chaque cellule intestinale au cours d'une période d'incubation inférieure ou égale à 5 minutes ;
d) incubation des souches dans un milieu nutritif et sélection des bactéries qui produisent au moins 3,5 mEq d'acide lactique pour 10¹⁰ UFC (Unités de Formation de la Colonie) à 37°C dans un temps de 24 heures ;
e) incubation des souches dans un milieu nutritif, et sélection des souches bactériennes qui prolifèrent au cours d'une durée de génération inférieure à 1 heure à 37°C.

8. Culture choisie parmi les cultures suivantes déposées à la collection CNCM de l'Institut Pasteur :
- Lactobacillus Paracasei, CNCM I-1687 ;
- Lactobacillus Paracasei, CNCM I-1688 ;
- Lactobacillus Salivarius, CNCM I-1794.

9. Préparation pour le traitement de troubles du système gastro-intestinal chez les humains, contenant une quantité efficace d'au moins une souche de bactéries lactiques telle que définie dans la revendication 8, en combinaison avec un support pharmaceutiquement ou physiologiquement acceptable.

10. Préparation selon la revendication 9, choisie parmi des compositions pharmaceutiques, des suppléments alimentaires et des produits alimentaires.

11. Préparation selon la revendication 10, pour une utilisation par voie orale.

12. Préparation selon la revendication 9, dans laquelle ladite souche de bactéries lactiques est sous forme lyophilisée.

13. Préparation selon la revendication 9, choisie parmi des compositions pharmaceutiques et des suppléments alimentaires, dans laquelle des lactobacilles sont combinées avec des excipients ou des additifs, choisis parmi les vitamines, les hydrocarbures et le dioxyde de silicium.

14. Préparation selon la revendication 9, contenant des vitamines B1 et B2, de la vitamine C et des mélanges de celles-ci, du saccharose, des maltodextrines et du dioxyde de silicium.

15. Utilisation d'une culture contenant une souche de bactéries lactiques telle que définie dans la revendication 8, pour la préparation d'un médicament pour le traitement des troubles du système gastro-intestinal chez les humains.

16. Utilisation selon la revendication 15, dans laquelle lesdits troubles sont choisis dans le groupe constitué par les diarrhées spécifiques et non spécifiques, la constipation, la colite, les flatulences, les brûlures d'estomac, la gastro-entérite, la stomatite aphteuse, et le syndrome de l'intestin irritable.
